# EUROPEAN PATENT SPECIFICATION

(11) **EP 1 082 290 B1**
(45) Date of publication and mention of the grant of the patent: **26.11.2003**
(21) Application number: 99918244.7
(22) Date of filing: 22.04.1999
(51) Int. Cl.: C07C 227/18, C07C 229/42

(54) **PROCESS FOR THE PREPARATION OF ACECLOFENAC**
VERFAHREN ZUR HERSTELLUNG VON ACECLOFENAC
PROCEDE DE PREPARATION D'ACECLOFENAC

(30) Priority: 28.04.1998 IE 980324
(43) Date of publication of application: 14.03.2001
(73) Proprietor: RUSSINSKY LIMITED, Cork (IE)
(72) Inventor: SCHICKANEDER, Helmut, South Terrace, Cork (IE); NIKOLOPOULOS, Aggelos, Model Farm Road, Cork (IE); MURPHY, Trevor, Ballinlough, Cork (IE)
(74) Representative: O'Brien, John Augustine
(86) International application number: IE9900028
(87) International publication number: WO99055660

(56) References cited:
- EP-A- 0 428 352
- EP-A- 0 600 395
- WO-A-92/16492
- CA-A- 2 111 169

## Description

### Introduction

The invention relates to a process for preparing non-steroidal anti-inflammatory drugs, to intermediates used in the process, and processes for preparing such intermediates.

Aceclofenac (formula III) is one example of a non-steroidal anti-inflammatory drug (NSAID) with properties similar to Diclofenac. The gastrointestinal tolerability of Aceclofenac is better than that of Diclofenac and other NSAIDs and it has a faster onset of action (Drugs Vol. 52(1), 113-124 [1996]).

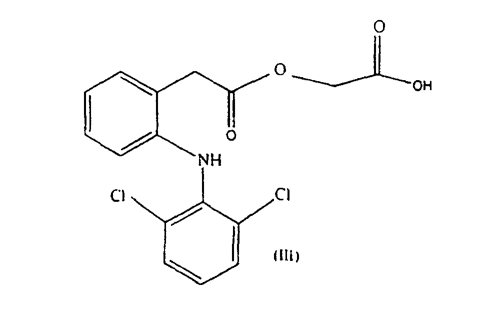

EP-A-119932 describes a process for preparing Aceclofenac by hydrogenation of benzyl-2-[(2,6-dichlorophenyl)amine] phenylacetoxyacetate with a palladium catalyst over a long period of time at severe reaction conditions. The 2-[(2,6-dichlorophenyl)amine] phenylacetoxyacetate is prepared by dissolving the corresponding phenylacetate in DMF and reacting with benzyl bromoacetate.

ES-A-2020146 describes the preparation of Aceclofenac by treating corresponding esters with iodine trimethylsilane which is prepared from chloromethylsilane and anhydrous sodium iodide in an inert atmosphere. Acetonitrile is used as the solvent.

CH-A-682747 describes a process for preparing Aceclofenac by acid hydrolysis of a 2-tetrahydropyranyl or 4-methoxy-4-tetrahydropyranyl ester. The esters are prepared by reacting the corresponding acetic acid with a corresponding haloacetate.

CA-A-2111169 describes phenylacetic acid derivatives and their salts. Sodium diclofenac is dissolved in DMF under a nitrogen atmosphere, the temperature is raised and *tert*.-butyl chloroacetate is added to yield *tert*.-butyl (2-(2,6-dichloroaniline)phenyl) acetoxyacetate.

There are a number of problems with conventional processes for preparing Aceclofenac. The yield of at least some of the steps is low, the reaction time is relatively high, hazardous reaction conditions and/or solvents are required and/or the use of dipolar aprotic solvents such as DMF causes difficulties in purification of the final product.

There is therefore a need for an improved process for preparing Aceclofenac which will overcome at least some of these problems and thereby provide a process which is economic and viable on a commercial scale.

### Statements of Invention

The invention provides a compound of formula I 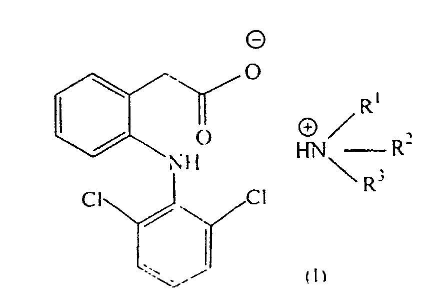 wherein R¹, R² and R³ are independently selected from one or more of ethyl and isopropyl.

In one embodiment the invention provides a compound of Formula I wherein each of R¹ to R³ are ethyl.

In another embodiment the invention provides a compound of Formula I wherein R¹ and R² are isopropyl and R³ is ethyl.

The invention also provides a process for preparing a compound of Formula I by reacting 2-[(2,6-Dichlorophenyl)amine]phenylacetic Acid (Diclofenac Acid) with an appropriate amine of the formula NR¹R²R³ wherein R¹, R² and R³ are as defined above.

The reaction may be carried out in a solvent selected from toluene, THF, acetone, MEK, MIBK, acetonitrile or a chlorinated solvent. The formation of adduct I is carried out at a temperature of from 0 to 100°C, preferably from 20 to 60°C. The amine may be triethylamine or diisopropylethylamine.

The invention also provides a process for preparing a compound of the Formula II 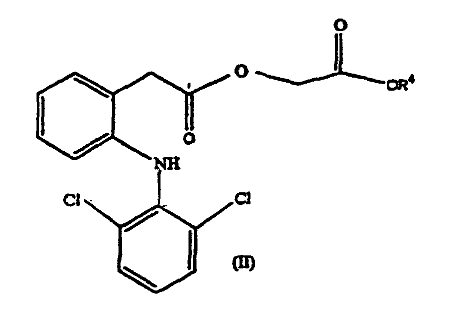 wherein R⁴ is a lower alkyl (C₁-C₄)
by reacting a compound of Formula I with an appropriate α-haloacetic acid ester, especially *tert*.-butyl-bromoacetate.

In this case the substituent R⁴ is preferably *tert*. Butyl.

Preferably the reaction is carried out at a temperature of from 0 to 100°C, most preferably 20 to 60°C.

A further embodiment of the invention provides a process for preparing a compound of the Formula III 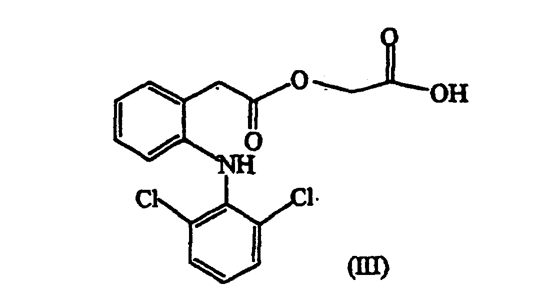 which comprises reacting a compound of the Formula I 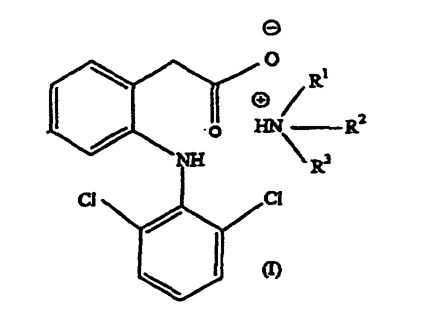 with an appropriate α-haloacetic acid to form a compound of the Formula II 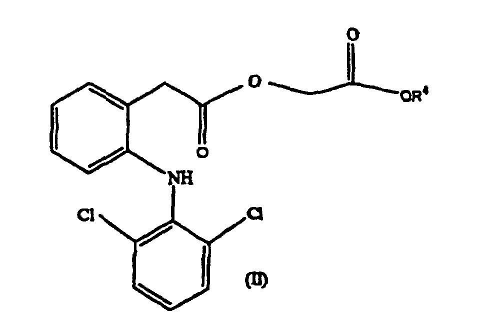 and subsequently treating a compound of the Formula II with a deprotecting agent to form a compound of Formula III.

One aspect of the invention provides for a compound of the Formula 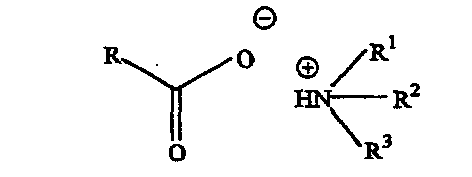 wherein R-COOH is an α-Arylpropanoic Acid NSAID.

Another aspect provides for a compound of the Formula 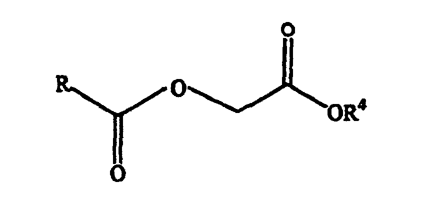 wherein R-COOH is an α-Arylpropanoic Acid NSAID and R⁴ is C₁ to C₄ alkyl.

A further aspect of the invention provides for a process for preparing a chain extended α-Arylpropanoic Acid with an appropriate amine of the formula NR¹R²R³ wherein R¹, R² and R³ are as defined above.

### Detailed description of the invention

We have found that compounds of the general Formula I are synthetically very . useful compounds, especially as intermediates for producing 2-[(2,6-Dichlorophenyl)-amine]phenylacetoxyacetic acid (Aceclofenac).

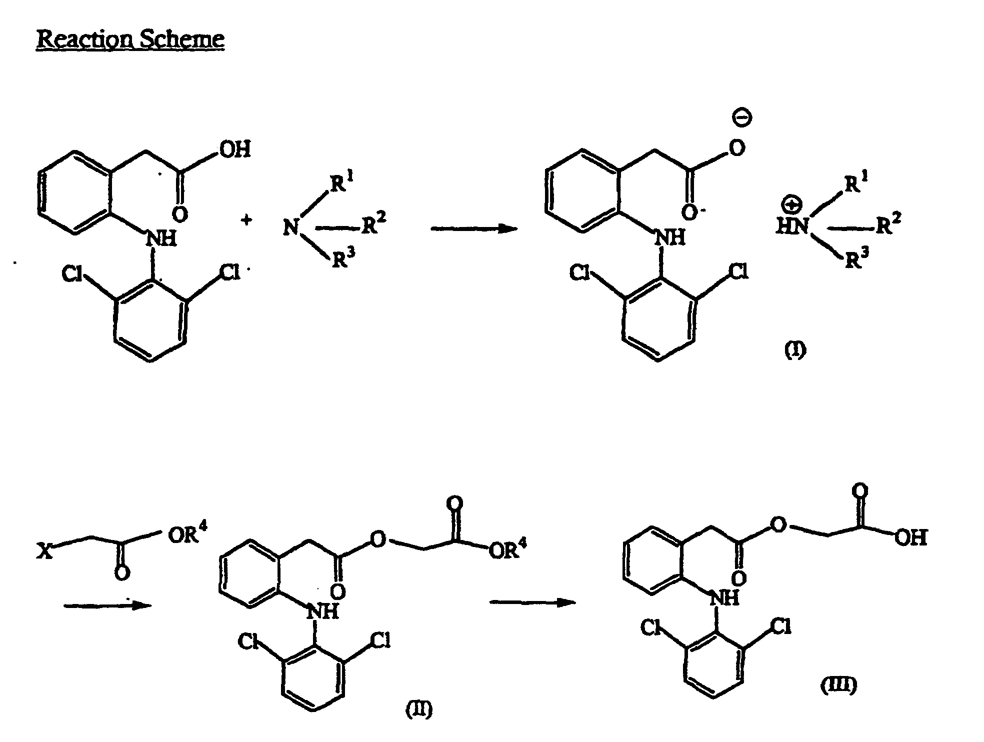

Compound I can be obtained in a simple process by reacting 2-[(2,6-Dichlorophenyl)amine]phenylacetic Acid - (Diclofenac Acid) with an amine NR¹R²R³. It was found that a variety of amines are suitable for the formation of adduct I. R¹, R² and R³ can independently selected from one or more of ethyl and isopropyl. The solvents are toluene, THF, acetone, MEK, MIBK, acetonitrile or a chlorinated solvent and the adduct formation is carried out under very mild conditions of 0°-100°C, preferably 20-60°C.

The salts of Formula I can be reacted without isolation and purification directly with various α-haloacetic acid esters to give compounds of type II. The halogen substituent X can be Cl or Br, preferably Br. Group R⁴ is a lower alkyl substituent C₁-C₄, preferably *tert*.-butyl. The reaction step is carried out in a temperature range of 20°-100°C, preferably 20°-60°C.

For the conversion of a compound of type II wherein R⁴ is *tert*.*-*butyl into 2-[(2,6-Dichlorophenyl)amine]phenylacetoxyacetic Acid (Aceclofenac), formic acid and trifluoroacetic acid are suitable. The reaction can be carried out under very mild conditions 0°-100°C, preferably 20°-60°C.

The procedure for the preparation of 2-[(2,6-dichlorophenyl)-amine]phenylacetoxyacetic Acid (Aceclofenac) is a major improvement compared to known methods as the process is very simple. The reaction sequence can be carried out in either separate reaction steps or in a one pot process. The reaction time is relatively short and the reaction process is carried out without the use of heavy metal catalysts and hydrogen and/or difficult solvents. The product is obtained in high overall yield in very high purity under extremely mild reaction conditions.

### Example 1

### Preparation of tert.-Butyl-2-[(2,6-dichlorophenyl)amine]phenylacetoxyacetate (method 1).

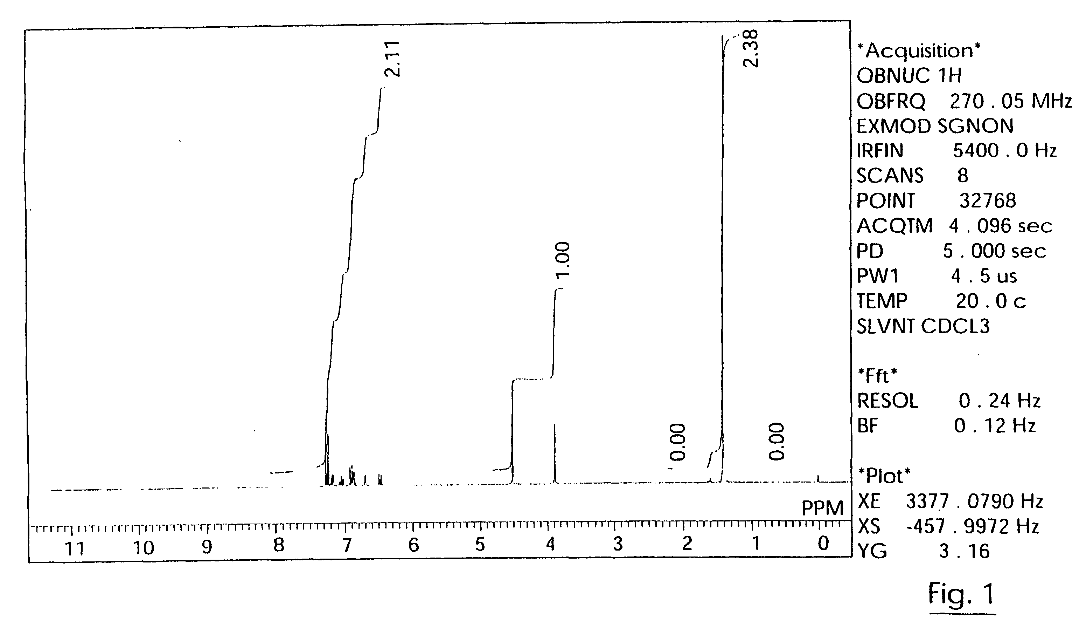

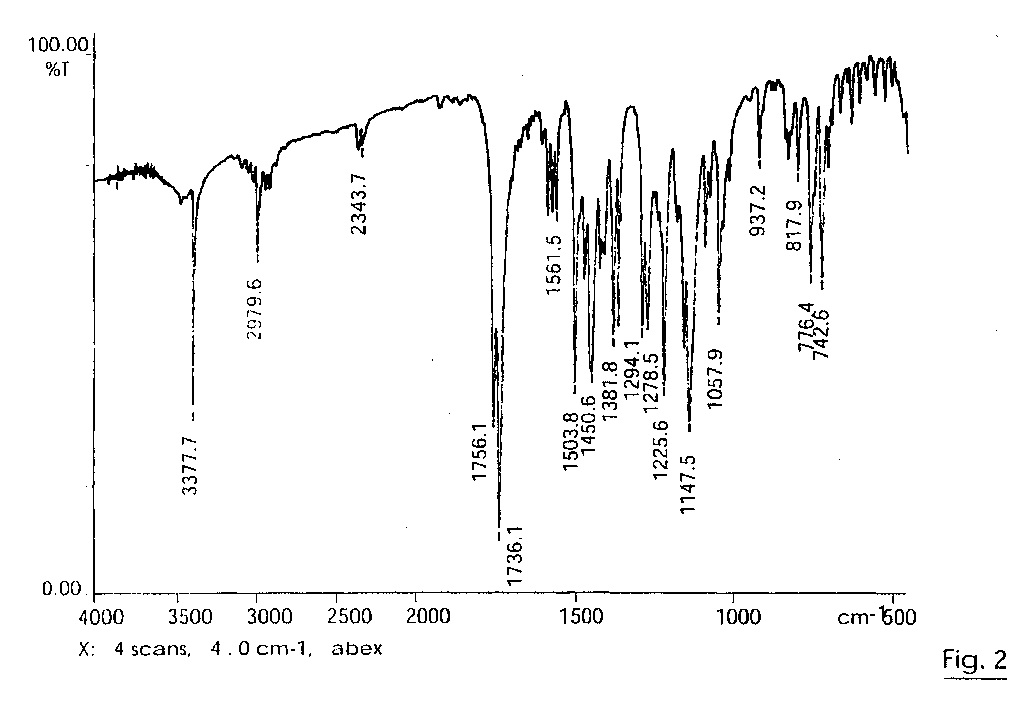

200 g (0.675. mol) of 2-[2,6-dichlorophenyl)amine]phenylacetic Acid were suspended in 800 ml of toluene at room temperature. 94 ml (0.675 mol) of triethylamine were added and the mixture was stirred until a clear solution was obtained. 109 ml (0.675 mol) of *tert*.-Butyl-bromoacetate were added. The mixture was heated to 40-60°C. After a reaction time of 3-4 hours 400 ml of water were added and the mixture was basified with 30% sodium hydroxide solution. The phases were separated and the organic layer was washed with water. The organic solvent was removed and the crude material purifird with Petroleum Ether. Yield 76%.
¹H-NMR spectrum as attached (figure 1).
IR spectrum as attached (figure 2).
Microanalysis.
calc.: C 58.54, H 5.12, N3.41;
found: C 58.70. H 5.32, N 3.30.

### Example 2

### Preparation of tert.-Butyl-2-[(2,6-dichlorophenyl)amine]phenylacetoxyacetate (method 2).

100 g (0.338 mol) of 2-[(2,6-Dichlorophenyl)amine]phenylacetic Acid were suspended in 300 ml of THF at room temperature. 58 ml (0.338 mol) of diisopropylethylamine were added and the mixture was stirred until a clear solution was obtained. 55 ml (0.338 mol) of *tert*.-Butyl-bromoacetate were added. The mixture was heated to 40-60°C. After a reaction time of 3-4 hours the mixture was basified with 30% sodium hydroxide solution. The phases were separated and the organic layer dried over sodium sulphate. The organic solvent was removed and the crude material purified with Petroleum Ether. Yield 64%.

### Example 3

### Preparation of Ammonium-2-[(2,6-dichlorophenyl)amine]phenylacetate.

100 g (0.338 mol) of 2-[(2,6-Dichlorophenyl)amine]phenylacetic Acid were added to 300 ml of aqueous ammonia (25-30%). The mixture was heated to reflux and then cooled to room temperature to precipitate the product. The solid was filtered off and dried under vacuum. Yield 96 g (90%).

### Example 4

### Preparation of 2-[(2,6-dichlorophenyl)amine]phenylacetoxyacetic Acid from tert.-Butyl-2-[(2,6-dichlorophenyl)amine]phenylacetoxyacetate (method 1).

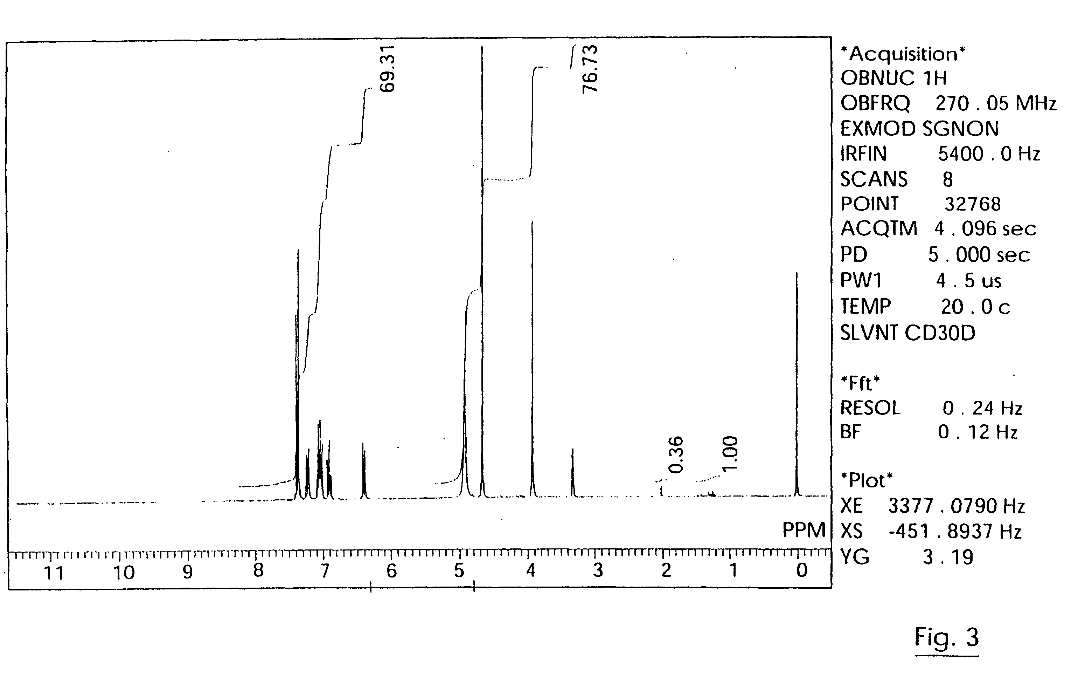

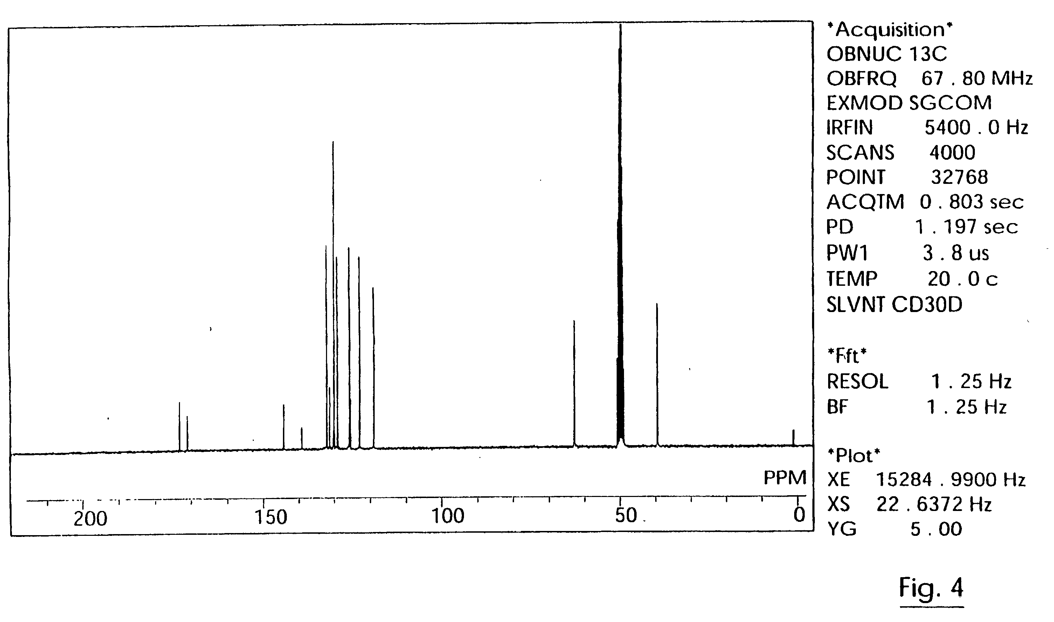

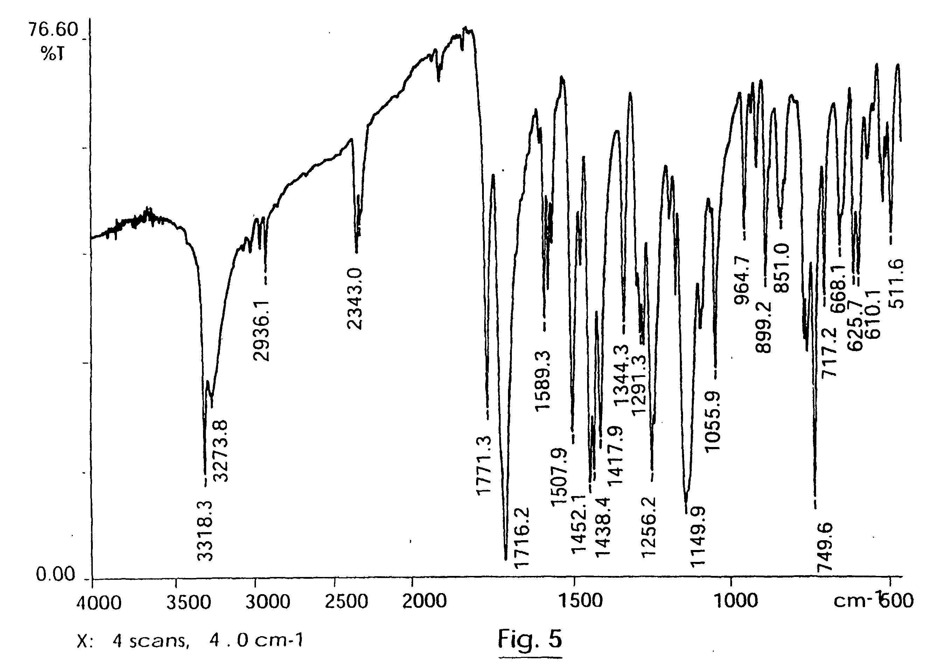

260g (0.634 mol) of *tert*.*-*Butyl-2-[(2,6-dichlorophenyl)amine]phenylacetoxyacetate were dissolved in 260 ml of formic acid. The mixture was stirred for 10-60 min, preferably 10-30 min at 20-80°C, preferably 50-60°C. The mixture was cooled and diluted with water to precipitate the product 2-[(2,6-dichlorophenyl)amine]phenylacetoxyacetic Acid. The crude material was recrystallised. Yield 204 g (91%).
Melting point 145°-149°C.
¹H-NMR spectrum as attached (figure 3).
¹³C-NMR spectrum as attached (figure 4).
IR spectrum as attached (figure 5).
Microanalysis:
calc.: C 54.26, H 3.67, N 3.95
found: C 54.40, H 3.69, N 3.88

### Example 5

### Preparation of 2-[(2,6-dichlorophenyl)amine]phenylacetoxyacetic Acid from tert.-Butyl-2-[(2,6-dichlorophenyl)amine]phenylacetoxyacetate (method 2).

10 g (0.024 mol) of *tert*.-Butyl-2-[(2,6-dichlorophenyl)amine]phenylacetoxyacetate were stirred in 50 ml of a 1:1 mixture of trifluoroacetic acid and dichloromethane at a temperature of 0-30°C, preferably 15-20°C for 10-70 min, preferably 20-40 min. The solvent was removed and the product 2-[(2,6-dichlorophenyl)amine]phenylacetoxyacetic Acid precipitated by adding water. The crude material was recrystallised. Yield 79%.

### Example 6

### Preparation of 2-[(2,6-dichlorophenyl)amine]phenylacetoxyacetic Acid in a one pot process.

800 g (2.70 mol) of 2-[(2,6-Dichlorophenyl)amine]phenylacetic Acid were suspended in 3.2 litres of toluene at room temperature. 273 g (2.70 mol) of triethylamine were added and the mixture stirred until a clear solution was obtained. 480 ml (2.96 mol) of *tert*.-Butyl-bromoacetate were added. The mixture was heated to 40-60°C. After a reaction time of 3-4 hours the mixture was basified with 30% sodium hydroxide solution. The phases were separated and the organic layer was washed with water. The organic solvent was removed and 1.4 litres of formic acid were added. The mixture was stirred at 50-60°C, cooled to room temperature after approximately 30 min and diluted with water. The product was filtered off and purified with toluene. Overall yield 832 g (87%).

It is anticipated that the invention may be applied to other α-Arylpropanoic Acid NSAID's. Analogous intermediates of structures I, II, and III above are also provided. The reaction scheme is analogous to that given above for Aceclofenac.

Some examples of α-Arylpropanoic Acid NSAID's to which the invention can be applied include the following:

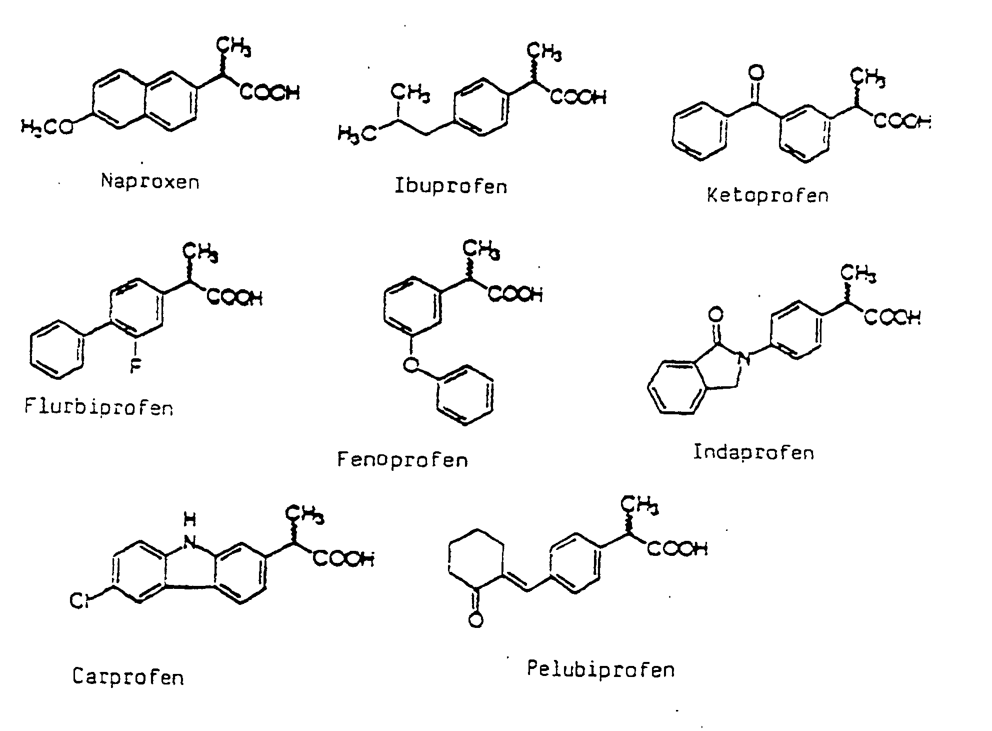

The invention is not limited to the embediments hereinbefore described which may be varied in detail.

## Claims

1. A compound of Formula I 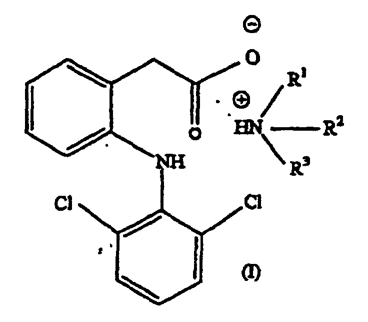 wherein R¹, R² and R³ are independently selected from one or more of ethyl and isopropyl.

2. A compound as claimed in claim 1 wherein each of R¹ to R³ are ethyl.

3. A compound as claimed in claim 1 wherein R¹ and R² are isopropyl and R³ is ethyl.

4. A process for preparing a compound of Formula I as defined in any of claims 1 to 3 by reacting 2-[(2,6-Dichlorophenyl)amine]phenylacetic Acid (Diclofenac Acid) with an appropriate amine of the formula NR¹R²R³ wherein R¹, R² and R³ are as defined in any of claims 1 to 3.

5. A process as claimed in claim 4 wherein the reaction is carried out in a solvent selected from toluene, THF, acetone, MEK, MIBK, acetonitrile or a chlorinated solvent.

6. A process as claimed in claim 4 or 5 wherein the adduct formation is carried out at a temperature of from 0 to 100°C.

7. A process as claimed in any of claims 4 or 6 wherein the adduct formation is carried out at a temperature of from 20 to 60°C.

8. A process as claimed in any of claims 4 to 7 wherein the amine is triethylamine.

9. A process as claimed in any of claims 4 to 7 wherein the amine is diisopropylethylamine.

10. A process for preparing a compound of the Formula II 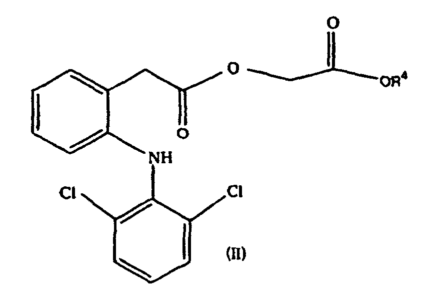 wherein R⁴ is a lower alkyl having from 1 to 4 carbon atoms,
by reacting a compound of Formula I as defined in any of claims 1 to 3 with an appropriate α-haloacetic acid ester.

11. A process as claimed in claim 10 wherein the halo group in α-haloacetic acid ester is Cl or Br.

12. A process as claimed in claim 10 or 11 wherein the halo group is Br.

13. A process as claimed in any of claims 10 to 12 wherein the α-haloacetic acid ester is *tert*.-Butyl-bromoacetate.

14. A process as claimed in any of claims 10 to 13 wherein R⁴ is tert. Butyl.

15. A process as claimed in any of claims 10 to 14 wherein the reaction is carried out at a temperature of from 0 to 100°C.

16. A process as claimed in any of claims 10 to 15 wherein the reaction is carried out at a temperature of from 20 to 60°C.

17. A process for preparing a compound of the Formula III 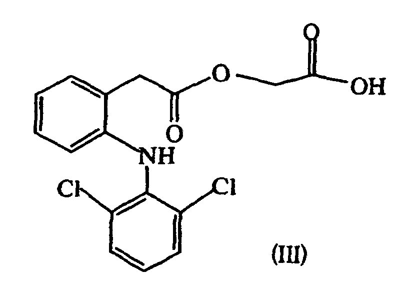 which comprises reacting a compound of the Formula I 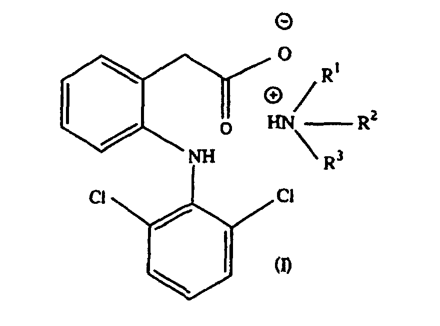 wherein R¹, R² and R³ are independently selected from one or more of ethyl and isopropyl,
with an appropriate α-haloacetic acid to form a compound of the Formula II 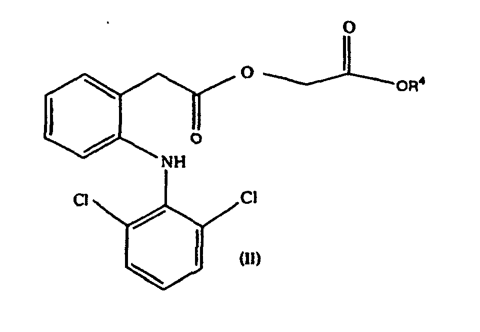 wherein R⁴ is a lower alkyl having from 1 to 4 carbon atoms,
and subsequently treating a compound of the Formula II with a deprotecting agent to form a compound of Formula III.

## Patentansprüche

1. Verbindung der Formel I 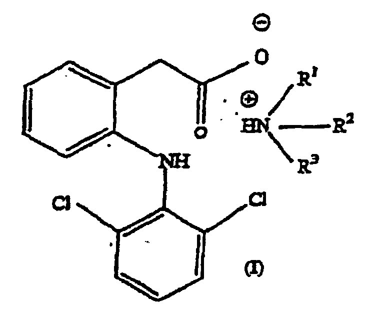 worin R¹, R² und R³ voneinander unabhängig aus einem oder mehreren von Ethyl und Isopropyl gewählt sind.

2. Verbindung nach Anspruch 1, worin R¹ bis R³ jeweils Ethyl sind.

3. Verbindung nach Anspruch 1, worin R¹ und R² Isopropyl sind und R³ Ethyl ist.

4. Verfahren zur Herstellung einer Verbindung der Formel I nach einem der Ansprüche 1 bis 3, wobei 2-[(2,6-Dichlorphenyl)amin]phenylessigsäure (Diclofenacsäure) mit einem geeigneten Amin der Formel NR¹R²R³, worin R¹, R² und R³ die in den Ansprüchen 1 bis 3 angegebenen Definitionen aufweisen, umgesetzt wird.

5. Verfahren nach Anspruch 4, worin die Reaktion in einem Lösungsmittel, das aus Toluol, THF, Aceton, MEK, MIBK, Acetonitril oder einem chlorierten Lösungsmittel gewählt ist, durchgeführt wird.

6. Verfahren nach Anspruch 4 oder 5, worin die Adduktbildung bei einer Temperatur von 0 bis 100°C durchgeführt wird.

7. Verfahren nach einem der Ansprüche 4 oder 6, worin die Adduktbildung bei einer Temperatur von 20 bis 60°C durchgeführt wird.

8. Verfahren nach einem der Ansprüche 4 bis 7, worin das Amin Triethylamin ist.

9. Verfahren nach einem der Ansprüche 4 bis 7, worin das Amin Diisopropylethylamin ist.

10. Verfahren zur Herstellung einer Verbindung der Formel II 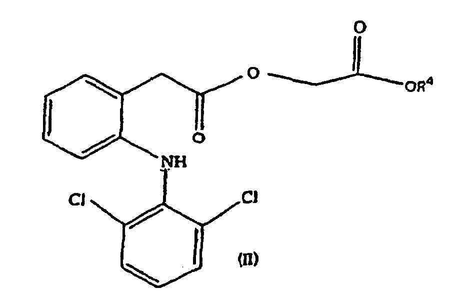 worin R⁴ ein niedrig molekulares Alkyl mit 1 bis 4 Kohlenstoffatomen ist, wobei eine Verbindung der Formel I, wie sie in einem der Ansprüche 1 bis 3 definiert ist, mit einem geeigneten α-Halogenessigsäureester umgesetzt wird.

11. Verfahren nach Anspruch 10, worin die Halogengruppe in dem α-Halogenessigsäureester Cl oder Br ist.

12. Verfahren nach Anspruch 10 oder 11, worin die Halogengruppe Br ist.

13. Verfahren nach einem der Ansprüche 10 bis 12, worin der α-Halogenessigsäureester *tert*.-Butylbromacetat ist.

14. Verfahren nach einem der Ansprüche 10 bis 13, worin R⁴ *tert*.-Butyl ist.

15. Verfahren nach einem der Ansprüche 10 bis 14, worin die Reaktion bei einer Temperatur von 0 bis 100°C durchgeführt wird.

16. Verfahren nach einem der Ansprüche 10 bis 15, worin die Reaktion bei einer Temperatur von 20 bis 60°C durchgefürht wird.

17. Verfahren zur Herstellung einer Verbindung der Formel III 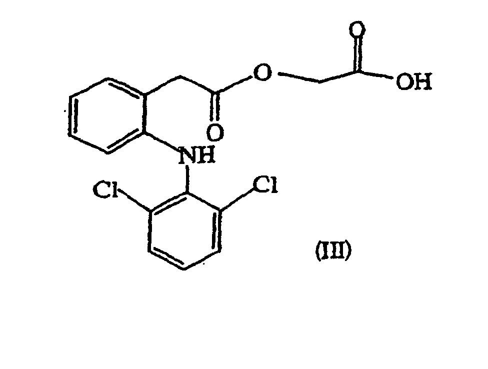 worin eine Verbindung der Formel I 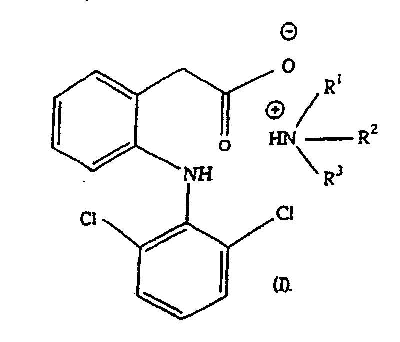 worin R¹, R² und R³ voneinander unabhängig aus einem oder mehreren von Ethyl und Isopropyl gewählt sind, mit einer geeigneten α-Halogenessigsäure unter Bildung einer Verbindung der Formel II 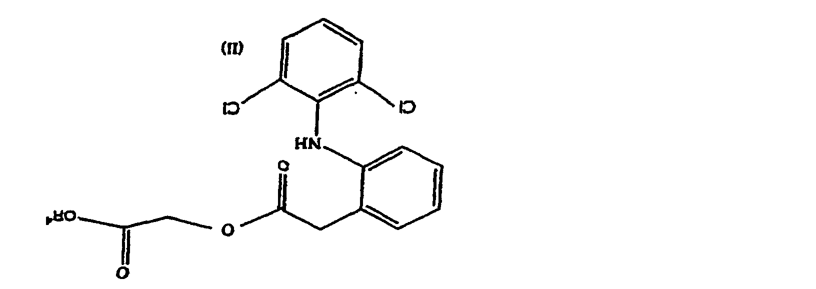 worin R⁴ ein niedrigmolekulares Alkyl mit 1 bis 4 Kohlenstoffatomen ist, umgesetzt wird und
anschließend eine Verbindung der Formel II mit einem Schutzgruppenentfernungsmittel unter Bildung einer Verbindung der Formel III behandelt wird.

## Revendications

1. Composé de Formule I 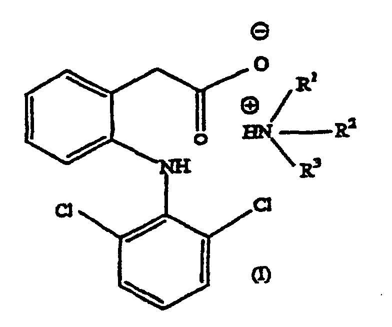 dans laquelle R¹, R² et R³ sont choisis indépendamment parmi un ou plusieurs parmi éthyle et isopropyle.

2. Composé selon la revendication 1, dans lequel chacun parmi R¹ à R³ est éthyle.

3. Composé selon la revendication 1, dans lequel R¹ et R² sont isopropyle et R³ est éthyle.

4. Procédé de préparation d'un composé de Formule I tel que défini selon l'une quelconque des revendications 1 à 3, par la réaction de l'acide 2-[(2,6-dichlorophényl)amine]phénylacétique (Acide Diclofénac) avec une amine appropriée de formule NR¹R²R³ dans laquelle R¹, R² et R³ sont tels que définis selon l'une quelconque des revendications 1 à 3.

5. Procédé selon la revendication 4, dans lequel la réaction est réalisée dans un solvant choisi parmi le toluène, le THF, l'acétone, la MEK, la MIBK, l'acétonitrile ou un solvant chloré.

6. Procédé selon la revendication 4 ou 5, dans lequel la formation du produit d'addition est réalisée à une température allant de 0 à 100°C.

7. Procédé selon l'une quelconque des revendications 4 ou 6, dans lequel la formation du produit d'addition est réalisée à une température allant de 20 à 60°C.

8. Procédé selon l'une quelconque des revendications 4 à 7, dans lequel l'amine est la triéthylamine.

9. Procédé selon l'une quelconque des revendications 4 à 7, dans lequel l'amine est la diisopropyléthylamine.

10. Procédé de préparation d'un composé de Formule II 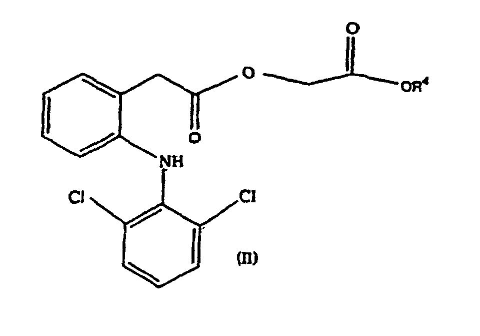 dans laquelle R⁴ est un alkyle inférieur ayant de 1 à 4 atomes de carbone,
en faisant réagir un composé de Formule I tel que défini selon l'une quelconque des revendications 1 à 3 avec un ester de l'acide α-halogénoacétique approprié.

11. Procédé selon la revendication 10, dans lequel le groupement halogéno dans l'ester de l'acide α-halogénoacétique est Clou Br.

12. Procédé selon la revendication 10 ou 11, dans lequel le groupement halogéno est Br.

13. Procédé selon l'une quelconque des revendications 10 à 12, dans lequel l'ester de l'acide α-halogénoacétique est le bromoacétate de tert-butyle.

14. Procédé selon l'une quelconque des revendications 10 à 13, dans lequel R⁴ est tert-butyle.

15. Procédé selon l'une quelconque des revendications 10 à 14, dans lequel la réaction est réalisée à une température allant de 0 à 100°C.

16. Procédé selon l'une quelconque des revendications 10 à 15, dans lequel la réaction est réalisée à une température allant de 20 à 60°C.

17. Procédé de préparation d'un composé de Formule III 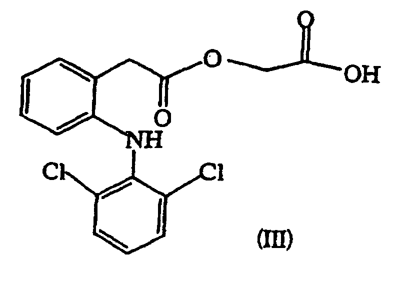 comprenant la réaction d'un composé de Formule I 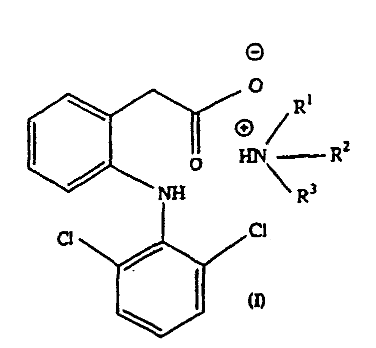 dans laquelle R¹, R² et R³ sont choisis indépendamment parmi un ou plusieurs parmi éthyle et isopropyle,
avec un acide α-halogénoacétique approprié pour former un composé de Formule II 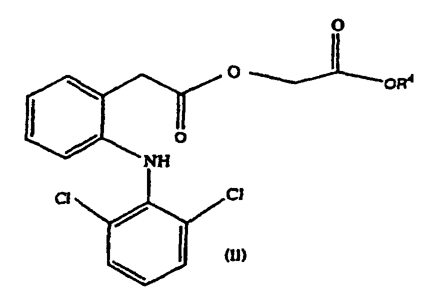 dans laquelle R⁴ est un alkyle inférieur ayant de 1 à 4 atomes de carbone,
et ensuite le traitement d'un composé de Formule II par un agent de déprotection pour former un composé de Formule III.
